# FASCICULE DE BREVET EUROPEEN

(11) **EP 0 837 706 B1**
(45) Date de publication et mention de la délivrance du brevet: **08.03.2000**
(21) Numéro de dépôt: 95925033.3
(22) Date de dépôt: 10.07.1995
(51) Int. Cl.: A61M 5/142

(54) **POMPE DE PERFUSION DE LIQUIDES MEDICAUX**
INFUSIONSPUMPE FÜR MEDIZINISCHE FLÜSSIGKEITEN
LIQUID DRUG INFUSION PUMP

(43) Date de publication de la demande: 29.04.1998
(73) Titulaire: COMPAGNIE DE DEVELOPPEMENT AGUETTANT, F-69007 Lyon (FR)
(72) Inventeur: FREZZA, Pierre, F-69390 Vourles (FR)
(74) Mandataire: Maureau, Philippe
(86) Numéro de dépôt international: FR9500922
(87) Numéro de publication internationale: WO9702852

(56) Documents cités:
- EP-A- 0 454 331
- FR-A- 2 689 014
- FR-A- 2 715 310
- US-A- 3 976 402
- US-A- 3 993 061
- US-A- 4 519 792
- US-A- 4 798 589

## Description

La présente invention a pour objet une pompe de perfusion de liquides médicaux.

La perfusion intraveineuse est aujourd'hui une technique essentielle de médication dans le cas de pathologies graves, comme le cancer par exemple.

L'efficacité des traitements est directement liée aux doses prescrites, et limitée par le niveau de toxicité des principes actifs. La précision de l'injection est donc un critère important dans le choix du matériel utilisé. En outre, il a été constaté que l'injection d'un principe actif devait, pour respecter les rythmes biologiques du patient, être effectuée de préférence à certaines heures de la journée, le débit d'injection pouvant être variable tout au cours de la période d'injection pour tenir compte des effets immédiats du principe actif sur l'organisme.

En outre, il a été constaté qu'il était possible, voire avantageux, d'associer plusieurs produits, dont certains peuvent être injectés simultanément à l'aide du même cathéter, et d'autres injectés par des voies différentes.

Une tendance de la médecine actuelle est d'évoluer vers des traitements au domicile du patient, ceci étant plus particulièrement vrai pour les pathologies lourdes telles que traitements du cancer et du S.I.D.A., qui exigent une technicité très importante en milieu hospitalier et un suivi au domicile du patient.

La plupart des matériels de perfusion existant actuellement sur le marché ne tiennent pas compte de cette évolution des soins vers le domicile du patient. Les pompes ambulatoires existantes sont généralement lourdes, encombrantes, et possèdent une autonomie limitée. Les pompes les plus utilisées sont des pompes péristaltiques, qui réalisent l'écrasement d'un tube en polymère ou en silicone pour assurer le déplacement du produit entre un réservoir et le cathéter d'alimentation du patient.

Ce type d'appareil présente l'inconvénient de cumuler des solutions techniques d'encombrement et de poids importants :

L'écrasement du tube exige une force importante qui est obtenue par un système à galets encombrant et lourd. Le moteur doit être dimensionné pour permettre d'obtenir ces forces d'écrasement, ce qui conduit à un poids important. La consommation en énergie du moteur étant importante, son alimentation doit être réalisée par une série de piles ou de batteries volumineuses et lourdes, qui constituent une contradiction avec le principe même du caractère ambulatoire de la pompe.

Une autre solution consiste à utiliser des dispositifs du type pousse-seringue, dans lesquels le piston d'une seringue contenant le produit à injecter est entraîné par un moteur pas à pas. Outre les inconvénients de poids et d'encombrement décrits précédemment, un tel système ne permet pas généralement une programmation, et ne peut convenir que pour la perfusion d'un volume limité de liquide.

Le document FR-A-2 689 014 au nom de la Demanderesse décrit une pompe de perfusion de liquides médicaux, à plusieurs canaux, c'est-à-dire susceptible de permettre la perfusion simultanée de plusieurs liquides médicaux, avec possibilité de mélange ou non de ces liquides à l'intérieur de la pompe, qui soit d'un très faible encombrement, et d'un poids réduit, tout en permettant une très grande précision du débit sur une plage de débits très large pouvant varier par exemple entre 1 millilitre par jour et 300 millilitres par heure, et qui offre une sécurité importante pour le patient.

La pompe décrite dans ce document, du type comprenant au moins un dispositif d'aspiration à cylindre et piston, d'un liquide dans un récipient et de refoulement de celui-ci dans une tubulure reliée au patient, est caractérisée en ce qu'elle comprend :
- une première pièce dans laquelle est ménagé au moins un cylindre de dosage qui, contenant un piston animé d'un mouvement alternatif, débouche dans une des faces de la pièce, perpendiculairement à cette face,
- une seconde pièce qui, prenant appui sur la face de la première pièce dans laquelle débouche chaque cylindre, comporte en regard de chaque cylindre, une cavité de diamètre supérieur à celui du cylindre correspondant, et communiquant avec un récipient d'alimentation en liquide,
- et une membrane étanche et déformable élastiquement obturant l'extrémité du cylindre opposée au piston, et dont l'autre face est située du côté de l'amenée du liquide à partir d'un récipient, cette membrane comportant au moins une ouverture pour le passage du liquide et permettant l'alimentation en liquide du cylindre lors du déplacement du piston dans un sens d'augmentation du volume du cylindre, tandis qu'elle assure l'isolement du cylindre vis-à-vis de l'alimentation lors du déplacement du piston en sens inverse, le liquide étant alors évacué du cylindre par au moins une lumière ménagée dans celui-ci à proximité de la membrane.

Dans le dispositif connu, la chambre de dosage est délimitée par une membrane déformable, enserrée entre deux parties du piston, l'une située à l'intérieur de la chambre de dosage, et l'autre située à l'extérieur de la chambre de dosage. Les deux parties du piston sont assemblées, par exemple par vissage à l'aide d'une vis traversant un orifice ménagé dans la membrane. Cette solution présente l'inconvénient qu'une étanchéité parfaite n'est pas réalisée entre le circuit de liquide et l'extérieur. En outre, le piston doit être accouplé au dispositif moteur, de façon à pouvoir être tiré et poussé. Dans la mesure où l'on souhaite démonter le moteur en séparant la partie moteur et programmation de la partie dosage, qui est une partie d'usure, il convient de mettre en oeuvre des moyens complexes, notamment par un accouplement magnétique grâce à une pastille ferromagnétique ménagée dans le piston. Toutefois, outre sa complexité, cette solution présente l'inconvénient d'un accrochage relativement aléatoire du piston vis-à-vis du moteur.

Le but de l'invention est de fournir une pompe du dernier type cité, dans laquelle l'étanchéité entre le liquide et l'extérieur soit réalisé de façon rigoureuse, et dans laquelle l'accouplement entre la partie moteur et le piston soit réalisé de façon simple et tout à fait sûre.

A cet effet, dans la pompe qu'elle concerne, qui est du type précité, le piston est recouvert sur sa face en bout et sur sa paroi latérale par une membrane élastique, montée en tension sur le piston, et fixée par son bord périphérique sur un support fixe indépendant du piston, les moyens d'entraînement du piston étant constitués par un dispositif exerçant seulement une force de poussée sur le piston dans le sens de pénétration du piston dans le cylindre, l'épaisseur de la membrane est supérieure au niveau de la face en bout du piston à celle au niveau de la paroi latérale de celui-ci, et la zone de plus forte épaisseur de la membrane possède, sur sa face tournée vers l'extérieur, un évidement de forme concave, sensiblement complémentaire de la forme convexe de la membrane délimitant le cylindre.

La membrane élastique recouvrant le piston assure une parfaite étanchéité du liquide vis-à-vis de l'extérieur en isolant parfaitement la chambre de dosage. La force de rappel élastique de la membrane s'exerce sur le piston et assure ainsi le mouvement de retour de celui-ci lorsqu'aucune action ne s'exerce plus sur lui. Il suffit donc de disposer de moyens d'entraînement qui exercent une poussée sur le piston dans le sens de pénétration dans le cylindre, le piston ayant tendance, sous l'effet de détente de la membrane, à reculer dès qu'il n'est plus poussé vers l'intérieur du cylindre. Il n'est donc pas nécessaire de prévoir des moyens positifs d'entraînement entre les moyens moteurs et le piston, un simple contact étant suffisant.

Cette forme de la membrane permet d'éviter le ménagement d'un volume mort entre celle-ci et la membrane supérieure, en position avancée du piston, ce qui facilite l'amorçage de la pompe.

Suivant une caractéristique de l'invention, la membrane élastique recouvrant le piston est réalisée en élastomère ou en latex.

Avantageusement, la membrane présente, à proximité de sa partie recouvrant la face avant du piston, un jonc périphérique assurant l'étanchéité vis-à-vis du cylindre. Cet agencement permet de maintenir constant le volume de liquide résiduel entre la chambre et la membrane, quelles que soient les pressions d'utilisation.

Selon une forme d'exécution de cette pompe, le dispositif d'entraînement de chaque piston comprend un disque d'axe orthogonal à l'axe du cylindre dont le bord prend appui sur l'extrémité libre du piston, et qui est entraîné en rotation autour d'un axe parallèle et décalé par rapport à son propre axe.

Avantageusement dans ce cas, le disque dont le bord prend appui contre l'extrémité libre du piston est fixé sur un disque, dont une face comporte un crantage coopérant avec un pignon calé sur l'arbre de sortie d'un moto-réducteur, les deux disques étant fixés l'un sur l'autre avec décalage de leurs axes respectifs.

Il est compréhensible que le disque en appui sur l'extrémité libre du piston possède un centre dont la position va varier vis-à-vis du centre du disque motorisé, par rotation autour du centre du disque motorisé. Il en résulte que le disque entraîné va pouvoir successivement exercer une poussée sur le piston, puis relâcher cette poussée pour permettre au piston de revenir en arrière sous l'effet de détente de la membrane.

Avantageusement, sur l'axe du disque motorisé est monté un mécanisme à roue libre empêchant le mouvement du disque dans le sens contraire au sens d'entraînement par le moteur, sous l'effet de l'effort de traction de la membrane, lorsque le système est à l'arrêt ou non alimenté.

Suivant une autre caractéristique de l'invention, le disque motorisé comporte des lumières périphériques destinées à être détectés par un codeur optique pour déterminer le volume de liquide transféré, ce codeur étant relié à un compteur d'impulsion lui-même relié à un comparateur, comparant les impulsions mesurées à une valeur de consigne, pour commander l'alimentation électrique du moteur.

Avantageusement, les lumières périphériques du disque motorisé sont disposées selon une fonction sinusoïdale pour tenir compte du décalage des axes des deux disques. Cette disposition relative des lumières périphériques permet de faire correspondre un volume constant à l'écartement entre deux lumières, bien que les lumières ne soient pas toutes équidistantes.

Afin de favoriser la douceur du fonctionnement de l'ensemble, le bord du disque prenant appui contre le piston est recouvert d'un roulement à billes.

Dans le cas où la pompe comprend un boîtier contenant les moyens de programmation et le moteur associé à chaque piston, et une cassette amovible assurant l'aspiration et le refoulement des différents liquides, chaque piston et la membrane qui le recouvre sont montés dans la cassette.

Le boîtier de commande et la cassette peuvent alors être assemblés de façon simple et rapide, par exemple par encliquetage.

De toute façon, l'invention sera bien comprise à l'aide de la description qui suit, en référence au dessin schématique annexé représentant, à titre d'exemple non limitatif, une forme d'exécution de cette pompe :
Figure 1 est une vue en perspective d'une pompe destinée à véhiculer quatre liquides distincts ;
Figure 2 en est une vue en coupe à échelle agrandie selon la ligne II-II de figure 1 ;

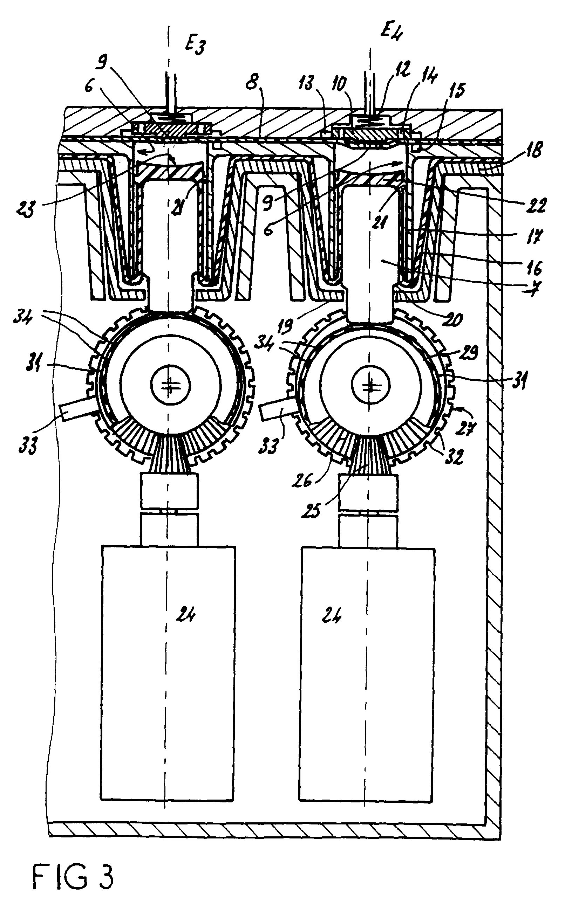

Figure 3 en est une vue partielle, en coupe longitudinale, selon la ligne III-III de figure 1, à échelle agrandie ;
Figure 4 est une vue de détail du dispositif de commande du mouvement.

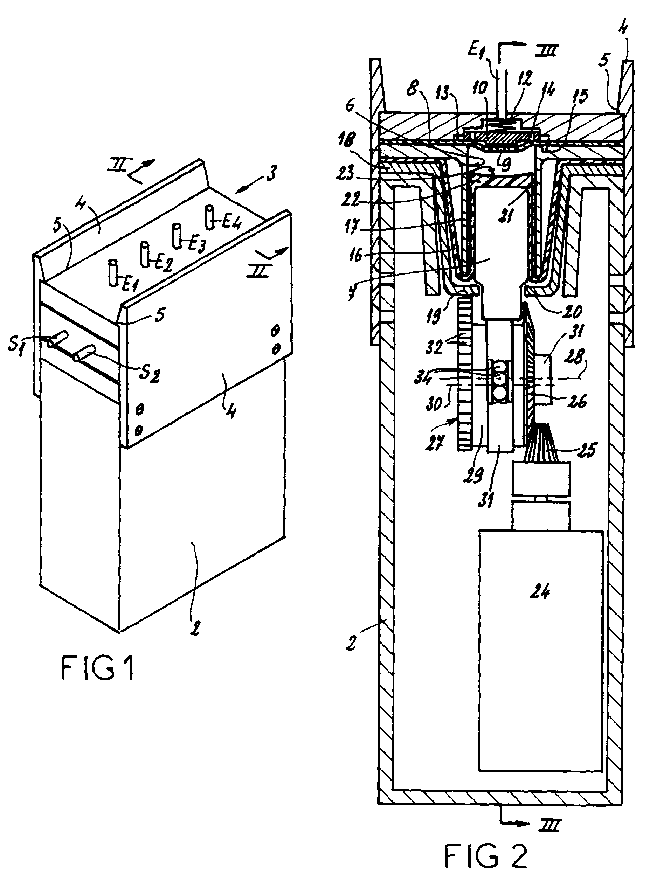

La pompe de perfusion, représentée à la figure 1, est destinée à véhiculer quatre liquides différents. Elle comprend un boîtier 2 contenant des moyens de programmation de l'écoulement de chacun des quatre liquides et de commande des pistons des dispositifs de dosage. Ce boîtier contient également des piles électriques pour l'alimentation des moteurs assurant le déplacement des pistons.

Sur ce boîtier est montée, de façon amovible, une cassette 3 qui correspond à la partie assurant l'aspiration de chaque liquide à l'intérieur d'un récipient constitué par exemple par une poche en matière synthétique, un flacon comportant une prise d'air ou une seringue, et le refoulement de ce liquide dans une tubulure reliée par un cathéter au corps du patient. Les admissions de liquides à l'intérieur de la cassette portent les références E1, E2, E3 et E4, tandis que deux sorties représentées portent les références S1 et S2. La fixation de la cassette 3 sur le boîtier 2 est réalisée par encliquetage, à l'aide de deux éléments 4 prolongeant les grandes faces du boîtier 2, qui comportent des épaulements 5 destinés à venir prendre appui sur la face supérieure de la cassette.

La structure de la cassette ne sera pas décrite en détail du fait qu'elle est déjà connue en soi, et correspond à l'agencement décrit dans le document FR-A-2 689 014.

Cette cassette comprend quatre cylindres 6 de dosage, dans chacun desquels est engagé un piston 7 susceptible d'un mouvement alternatif. L'extrémité de la chambre du cylindre 6 opposée à celle par laquelle est actionné le piston est obturée par une membrane 8 comportant un orifice central 9 et contre laquelle est appuyé un disque 10 soumis à l'action d'un ressort 12, logé dans une chambre 13 située dans le prolongement du cylindre 6. Le disque 10 possède des perçages axiaux 14 pour permettre le passage de liquide depuis l'orifice d'entrée E1 en direction de la membrane 8. De l'autre côté de la membrane 8 est ménagée une lumière 15 d'évacuation du liquide communiquant avec une sortie S1.

Selon la caractéristique essentielle de l'invention, le piston est recouvert sur sa face en bout et sur sa paroi latérale par une membrane élastique 16 montée en tension sur le piston et fixée par son bord périphérique sur un support fixe indépendant du piston.

Dans la forme d'exécution représentée au dessin, la membrane 16 s'étend sur la face intérieure de la pièce 17 délimitant le cylindre, remonte le long de l'extérieur de cette pièce 17 tubulaire, et est pincée mécaniquement entre un retour horizontal de la pièce 17, et un retour horizontal d'une pièce 18 entourant la pièce 17. Il est à noter que cette pièce 18 possède, à son extrémité inférieure, un retour 19 formant une butée empêchant la sortie du piston 7 qui possède à cet effet un épaulement 20. En conséquence, le piston 7 est associé à la cassette 3 et ne peut être dissocié de cette dernière lorsqu'une cassette est séparée d'un boîtier 2.

La membrane 16 présente, à proximité de sa partie recouvrant la face avant du piston, un jonc périphérique 21 assurant l'étanchéité vis-à-vis du cylindre dans lequel est monté le piston.

Il doit être noté que la membrane 16 comporte, au niveau de la face en bout du piston 7, une surépaisseur 22 dans laquelle est ménagé un évidement 23 de forme concave, sensiblement complémentaire de la forme convexe de la membrane 8 délimitant le cylindre de dosage. La membrane élastique 16 est réalisée en élastomère ou en latex.

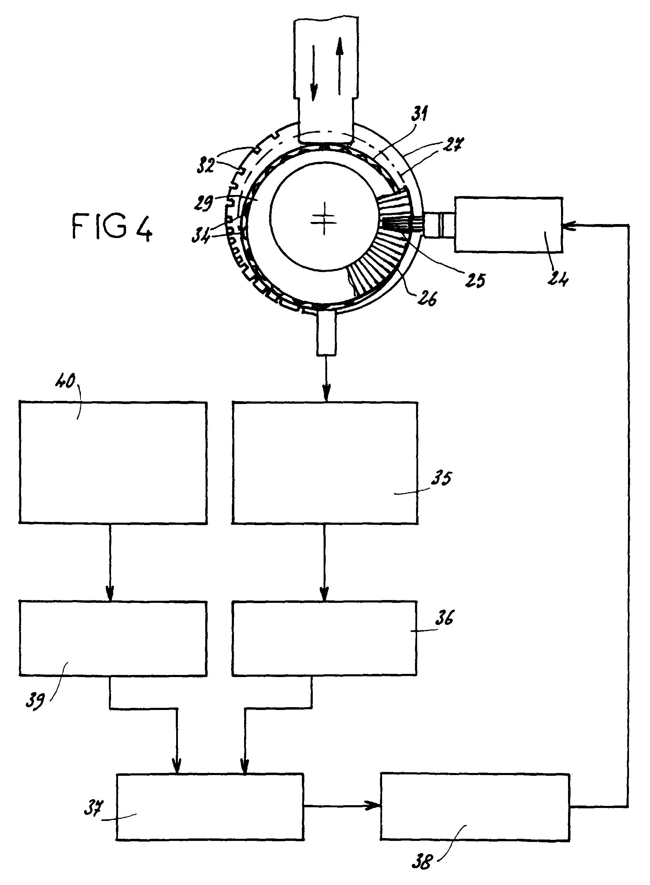

Le dispositif d'entraînement de chaque piston comprend un moteur électrique 24, dont l'arbre de sortie est équipé d'un pignon conique 25 engrenant avec la denture 26 d'un disque 27 dont l'axe 28 est orthogonal à l'axe du piston. Sur ce disque 27 est calé un disque 29, dont l'axe 30 est parallèle à l'axe 28 du disque 27 mais décalé par rapport à cet axe. Sur l'axe du disque motorisé 27, est monté un mécanisme à roue libre 31 empêchant toute rotation de ce disque dans un sens inverse à celui fourni par le moteur 24. Le disque 27 présente également des lumières périphériques 32 réparties sur sa périphérie, qui sont repérées au fur et à mesure de la rotation du disque par un codeur 33. Le disque excentré 29 est équipé sur sa périphérie d'un roulement à billes 34 dont la cage 31 prend appui sur l'extrémité basse du piston 7. Lors de la rotation du disque 27, l'excentration du disque 29 se traduit successivement par une poussée du piston 8, puis une suppression de cette poussée, situation dans laquelle le piston tend à augmenter le volume du cylindre de dosage 6, compte tenu du rappel élastique exercé par la membrane 16. Les lumières 32 sont ménagées selon une fonction sinusoïdale pour que l'espace entre deux lumières corresponde toujours au même volume malgré l'excentration du disque 29 de commande du piston. La figure 4 représente l'asservissement du moteur 24 à partir du codeur 33. Le codeur 33 est relié par l'intermédiaire d'un boîtier 35 à un compteur d'impulsions 36. Ce compteur d'impulsions fournit des informations à un comparateur 37, qui reçoit également des informations d'une horloge 38 et d'un compteur théorique d'impulsions 39. Ce comparateur agit sur un bloc 40 d'alimentation du moteur 24.

Il ressort de ce qui précède qu'une parfaite étanchéité entre chaque liquide à doser et l'extérieur est assurée par la membrane 16 disposée dans chaque cylindre de dosage. En outre, la fonction moteur de chaque membrane 16, qui assure le retour du piston lorsqu'aucune action n'est exercée sur lui, permet la mise en oeuvre d'un dispositif d'entraînement simple, qui consiste simplement à pousser le piston, sans nécessiter un accouplement positif entre les moyens d'entraînement et le piston.

Comme il va de soi, l'invention ne se limite pas à la seule forme d'exécution de ce dispositif, décrite ci-dessus à titre d'exemple, elle en embrasse au contraire toutes les variantes. C'est ainsi notamment que le dispositif d'alimentation, en liquide, de chaque cylindre, et les éléments associés à la membrane fermant celui-ci, pourraient être différents, sans que l'on sorte pour autant du cadre de l'invention.

## Revendications

1. Pompe de perfusion de liquides médicaux, comprenant au moins un dispositif d'aspiration à cylindre (6) et piston (7), d'un liquide dans un récipient, et de refoulement de celui-ci dans une tubulure reliée au patient, dans lequel l'extrémité de chaque cylindre (6) opposée à celle par laquelle est actionné le piston (7) est obturée par une face d'une membrane (8) étanche et élastique, dont l'autre face est située du côté de l'amenée du liquide à partir d'un récipient, cette membrane (8) comportant au moins une ouverture (9) pour le passage du liquide et permettant l'alimentation en liquide du cylindre (6) lors du déplacement du piston (7) dans un sens d'augmentation du volume du cylindre, tandis qu'elle assure l'isolement du cylindre (6) vis-à-vis de l'alimentation lors du déplacement du piston (7) en sens inverse, le liquide étant alors évacué du cylindre par au moins une lumière (15) ménagée dans celui-ci à proximité de la membrane (8), caractérisée en ce que le piston (7) est recouvert sur sa face en bout et sur sa paroi latérale par une membrane élastique (16), montée en tension sur le piston, et fixée par son bord périphérique sur un support fixe (18, 20) indépendant du piston, en ce que l'entraînement du piston est réalisé par un dispositif exerçant seulement une force de poussée sur le piston dans le sens de pénétration du piston dans le cylindre, en ce que l'épaisseur de la membrane (16) est supérieure au niveau de la face en bout du piston à celle au niveau de la paroi latérale de celui-ci, et en ce que la zone (22) de plus forte épaisseur de la membrane (16) possède, sur sa face tournée vers la membrane (8) délimitant le cylindre, un évidement (23) de forme concave, sensiblement complémentaire d'une zone de la forme convexe de la membrane (8) délimitant le cylindre.

2. Pompe selon la revendication 1, caractérisée en ce que la membrane élastique (16) recouvrant le piston (7) est réalisée en élastomère ou en latex.

3. Pompe selon l'une quelconque des revendications 1 et 2, caractérisée en ce que la membrane (16) présente, à proximité de sa partie recouvrant la face avant du piston (7), un jonc périphérique assurant l'étanchéité vis-à-vis du cylindre.

4. Pompe selon l'une quelconque des revendications 1 à 3, caractérisée en ce que le dispositif d'entraînement de chaque piston (7) comprend un disque (29) d'axe orthogonal à l'axe du cylindre dont le bord prend appui sur l'extrémité libre du piston (7), et qui est entraîné en rotation autour d'un axe (30) parallèle et décalé par rapport à son propre axe.

5. Pompe selon la revendication 4, caractérisée en ce que le disque (29) dont le bord prend appui contre l'extrémité libre du piston (7) est fixé sur un disque (27), dont une face comporte un crantage (26) coopérant avec un pignon (25) calé sur l'arbre de sortie d'un moto-réducteur (24), les deux disques (27, 29) étant fixés l'un sur l'autre avec décalage de leurs axes respectifs (28, 30).

6. Pompe selon la revendication 5, caractérisée en ce que sur l'axe du disque motorisé (27) est monté un mécanisme à roue libre empêchant le mouvement du disque (27) dans le sens contraire au sens d'entraînement par le moteur, sous l'effet de l'effort de traction de la membrane, lorsque le système est à l'arrêt ou non alimenté.

7. Pompe selon la revendication 5, caractérisée en ce que le disque motorisé (27) comporte des lumières périphériques (32) destinées à être détectés par un codeur optique (33) pour déterminer le volume de liquide transféré, ce codeur (33) étant relié à un compteur d'impulsions (36) lui-même relié à un comparateur (37), comparant les impulsions mesurées à une valeur de consigne, pour commander l'alimentation électrique du moto-réducteur (24).

8. Pompe selon la revendication 7, caractérisée en ce que les lumières périphériques (32) du disque motorisé sont disposées selon une fonction sinusoïdale pour tenir compte du décalage des axes des deux disques.

9. Pompe selon la revendication 4, caractérisée en ce que le bord du disque (29) prenant appui contre le piston est recouvert d'un roulement à billes (34).

10. Pompe selon l'une quelconque des revendications 1 à 8, comprenant un boîtier (2) contenant les moyens de programmation et le moto-réducteur (24) associé à chaque piston (7), et une cassette amovible (3) assurant l'aspiration et le refoulement des différents liquides, caractérisée en ce que chaque piston (7) et la membrane (16) qui le recouvre sont montés dans la cassette (3).

## Claims

1. Pump for the infusion of medical liquids, comprising at least one device with a cylinder (6) and a piston (7) for aspirating a liquid from a receptacle and for forcing this into a tube connected to the patient, wherein the end of each cylinder (6) opposite that through which the piston (7) is actuated is closed by one face of a leaktight and flexible membrane (8), the other face of which is situated on the side at which the liquid is brought in from a receptacle, this membrane (8) including at least one opening (9) for the passage of the liquid and enabling the cylinder (6) to be supplied with liquid when the piston (7) moves in a direction increasing the volume of the cylinder, while it ensures the isolation of the cylinder (6) from the supply when the piston (7) moves in the opposite direction, the liquid being then evacuated from the cylinder through at least one opening (15) provided therein close to the membrane (8), characterized in that the piston (7) is covered on its end face and on its side wall with an elastic membrane (16), mounted in tension on the piston, and fixed by its peripheral edge onto a fixed support (18, 20) independent of the piston, in that the piston is driven by a device exerting a thrusting force on the piston only in the direction of penetration of the piston into the cylinder, in that the thickness of the membrane (16) is greater in the region of the end face of the piston than in the region of its side wall, and in that the zone (22) having the greatest thickness of the membrane (16) possesses, on its face turned towards the membrane (8) delimiting the cylinder, a recess (23) having a concave form, substantially complementary to a zone with a convex form of the membrane (8) delimiting the cylinder.

2. Pump according to claim 1, characterized in that the elastic membrane (16) covering the piston (7) is made of an elastomer or a latex.

3. Pump according to either of claims 1 or 2, characterized in that the membrane (16) has, in the vicinity of its part covering the front face of the piston (7), a peripheral bead ensuring leaktightness to the cylinder.

4. Pump according to any one of claims 1 to 3, characterized in that the device for driving each piston (7) includes a disc (29) with its axis orthogonal to the axis of the cylinder, the edge of which rests on the free end of the piston (7), and which is driven in rotation about an axis (30) which is parallel and offset with respect to its own axis.

5. Pump according to claim 4, characterized in that the disc (29) of which the end rests against the free end of the piston (7) is fixed onto a disc (27), one face of which includes a serration (26) cooperating with a pinion (25) wedged onto the output shaft of a geared motor (24), both discs (27, 29) being fixed one on the other with their respective axes (28, 30) offset.

6. Pump according to claim 5, characterized in that a free-wheel mechanism is mounted on the axle of the motorised disc (27) preventing the disc (27) from moving in the opposite direction to the direction of drive by the motor, under the effect of the tensile force of the membrane, when the system is at rest or not supplied.

7. Pump according to claim 5, characterized in that the motorised disc (27) includes peripheral openings (32) designed to be detected by an optical encoder (33) in order to determine the volume of liquid transferred, this encoder (33) being connected to a pulse counter (36), itself connected to a comparator (37), comparing the pulses measured with a reference value, so as to control the electricity supply to the geared motor (24).

8. Pump according to claim 7, characterized in that the peripheral openings (32) of the motorised disc are arranged according to a sinusoidal function so as to take account of the offset between the axes of the two discs.

9. Pump according to claim 4, characterized in that the edge of the disc (29) resting against the piston is covered with a ball race (34).

10. Pump according to any one of claims 1 to 8, including a box (2) containing the programming means and the geared motor (24) associated with each piston (7), and a detachable cassette (3) ensuring aspiration and filling of different liquids, characterized in that each piston (7) and the membrane (16) which covers it are mounted in the cassette (3).

## Patentansprüche

1. Infusionspumpe für medizinische Flüssigkeiten, umfassend wenigstens eine Vorrichtung mit einem Zylinder (6) und einem Kolben (7) zum Ansaugen einer Flüssigkeit in einen Behälter und zum Verdrängen derselben in eine mit dem Patienten verbundene Leitung, bei der das Ende jedes Zylinders (6), das demjenigen entgegengesetzt ist, an dem der Kolben (7) betätigt wird, durch eine Fläche einer dichten und elastischen Membran (8) verschlossen ist, deren andere Fläche an der Seite der Flüssigkeitszufuhr aus einem Behälter angeordnet ist, wobei diese Membran (8) wenigstens eine Öffnung (9) für das Durchfließen der Flüssigkeit umfaßt, die die Versorgung des Zylinders (6) mit Flüssigkeit bei der Verlagerung des Kolbens (7) in einer Richtung der Vergrößerung des Zylindervolumens erlaubt, wohingegen sie die Isolierung des Zylinders (6) gegen die Versorgung bei der Verlagerung des Kolbens (7) in umgekehrter Richtung gewährleistet, wobei die Flüssigkeit dann aus dem Zylinder durch wenigstens eine Öffnung (15) abgeführt wird, die in ihm in der Nähe der Membran (8) vorgesehen ist, dadurch gekennzeichnet, daß der Kolben (7) an seiner Endfläche und an seiner Seitenwand durch eine unter Spannung auf dem Kolben angebrachte elastische Membran (16) bedeckt ist, die an ihrem Umfangsrand auf einem vom Kolben unabhängigen festen Träger (18, 20) befestigt ist, daß der Antrieb des Kolbens durch eine Vorrichtung erfolgt, die nur eine Druckkraft auf den Kolben in der Richtung des Eindringens des Kolbens in den Zylinder ausübt, daß die Dicke der Membran (16) im Bereich der Endfläche des Kolbens größer als im Bereich der Seitenwand desselben ist, und daß der Bereich (22) größter Dicke der Membran (16) an ihrer Fläche, die zu der den Zylinder begrenzenden Membran (8) hinzeigt, eine Ausnehmung (23) mit konkaver Gestalt aufweist, die im wesentlichen komplementär zu einem konvex gestalteten Bereich der den Zylinder begrenzenden Membran (8) ist.

2. Pumpe nach Anspruch 1, dadurch gekennzeichnet, daß die den Kolben (7) bedeckende elastische Membran (16) aus Elastomer oder aus Latex gefertigt ist.

3. Pumpe nach Anspruch 1 oder 2, dadurch gekennzeichnet, daß die Membran (16) in der Nähe ihres die Vorderfläche des Kolbens (7) bedeckenden Teils einen Umfangsring aufweist, der die Abdichtung gegen den Zylinder sicherstellt.

4. Pumpe nach einem der Ansprüche 1 bis 3, dadurch gekennzeichnet, daß die Antriebsvorrichtung jedes Kolbens (7) eine Scheibe (29) mit einer zur Zylinderachse orthogonalen Achse umfaßt, deren Rand am freien Ende des Kolbens (7) anliegt, und die zur Drehung um eine Achse (30) angetrieben ist, welche parallel und bezüglich ihrer eigenen Achse versetzt verläuft.

5. Pumpe nach Anspruch 4, dadurch gekennzeichnet, daß die Scheibe (29), deren Rand am freien Ende des Kolbens (7) anliegt, auf einer Scheibe (27) befestigt ist, deren eine Fläche eine Verzahnung (26) umfaßt, die mit einem auf der Ausgangswelle eines Getriebemotors (24) festgesetzten Ritzel (25) zusammenwirkt, wobei die beiden Scheiben (27, 29) aufeinander mit Versatz ihrer jeweiligen Achsen (28, 30) befestigt sind.

6. Pumpe nach Anspruch 5, dadurch gekennzeichnet, daß auf der Achse der motorisierten Scheibe (27) ein Freilaufmechanismus angebracht ist, der die Bewegung der Scheibe (27) in der zur Richtung des Motorantriebs entgegengesetzten Richtung unter der Wirkung der Zugkraft der Membran verhindert, wenn das System angehalten oder ausgeschaltet ist.

7. Pumpe nach Anspruch 5, dadurch gekennzeichnet, daß die motorisierte Scheibe (27) Umfangsöffnungen (32) aufweist, die dazu ausgelegt sind, von einem optischen Codierer (33) zur Bestimmung des transportierten Flüssigkeitvolumens erfaßt zu werden, wobei dieser Codierer (33) mit einem Pulszähler (36) verbunden ist, der wiederum mit einem Komparator (37) verbunden ist, welcher die gemessenen Pulse mit einem Sollwert vergleicht, um die elektrische Versorgung des Getriebemotors (24) zu steuern.

8. Pumpe nach Anspruch 7, dadurch gekennzeichnet, daß die Umfangsöffnungen (32) der motorisierten Scheibe gemäß einer Sinusfunktion angeordnet sind, um den Versatz der Achsen der beiden Scheiben zu berücksichtigen.

9. Pumpe nach Anspruch 4, dadurch gekennzeichnet, daß der Rand der am Kolben anliegenden Scheibe (29) mit einem Kugellager (34) bedeckt ist.

10. Pumpe nach einem der Ansprüche 1 bis 8, umfassend ein Gehäuse (2), das die Programmiermittel und den Getriebemotor (24) enthält, der dem jeweiligen Kolben (7) zugeordnet ist, sowie eine abnehmbare Kassette (3), die das Ansaugen und Verdrängen der verschiedenen Flüssigkeiten gewährleistet, dadurch gekennzeichnet, daß der jeweilige Kolben (7) und die ihn bedeckende Membran (16) in der Kassette (3) angebracht sind.
